# EUROPEAN PATENT APPLICATION

(11) **EP 4 336 187 A1**
(43) Date of publication of application: **13.03.2024**
(21) Application number: 22799088.4
(22) Date of filing: 03.05.2022
(51) Int. Cl.: G01N 33/68

(54) **HER2 VACCINE COMPOSITION**

(30) Priority: 04.05.2021 US 202163183869 P
(71) Applicant: Aston Sci. Inc., Seoul 06193 (KR)
(72) Inventor: DISIS, Mary L, Renton, Washington 17850 (US)
(74) Representative: ZBM Patents - Zea, Barlocci & Markvardsen
(86) International application number: PCT/KR2022/006317
(87) International publication number: WO 2022/235045

(57) **Abstract**

The present disclosure relates to a method for predicting reactivity of a HER2-ICD DNA vaccine composition, that is, the acquisition of immunogenicity and the therapeutic efficacy thereof, by measuring immunogenicity against a HER2-ICD antigen before vaccination. Additionally, by using the method for predicting reactivity, according to the present disclosure, a DNA vaccination target may be selected.

## Description

### Technical Field

This application claims the benefit of priority based on US Patent Application No. 63/183,869, filed May 04, 2021, the disclosure of which is incorporated herein by reference in its entirety.

The present disclosure relates to a HER2 vaccine composition.

### Background Art

Breast cancer is the most frequently diagnosed cancer in women, and is the most common cause of cancer-related death, with an increasing incidence and a gradually decreasing age of onset.

In the past 20 years, in the treatment of breast cancer, Disease Free Survival (DFC) has been significantly improved compared to other cancer types, through various targeted therapies and diagnostic methods. For example, in addition to traditional cancer treatments such as surgical operation and chemotherapy such as paclitaxel or radiotherapy, various targeted therapeutic agents are used to treat breast cancer or slow down the progression of breast cancer, such as the use of estrogen receptor (ER)-targeted therapeutic agents such as tamoxifen which inhibit the activation of ERs, for ER-positive breast cancer patients, and the use of HER2-targeted antibodies such as trastuzumab for HER2-positive patients.

However, despite advances in therapies for the treatment of breast cancer, since a great number of patients often die from metastasis or recurrence, there have been attempts to reduce breast cancer recurrence and increase overall survival (OS) or disease free survival (DFS).

As an attempt to prevent the metastasis or recurrence of breast cancer, research on how to discover biomarkers for breast cancer stem cells and target them for treatment is ongoing, and at the same time, immunotherapies using vaccines against breast cancer-specific antigens are being studied. Cancer vaccines are designed to activate cytotoxic T cells so that the activated T cells can recognize and act against specific types of cancer cells or induce the production of antibodies that bind to antigens present on the surfaces of cancer cells.

Vaccine research has been extensively conducted over the past few years to treat cancer or prevent recurrence, but there are no successful cases yet, except for the prostate cancer vaccine sipuleucel-T (Provenge^{®}). Regarding this low success rate, even if an antigen is specifically expressed in a specific type of tumor cell, depending on each individual, the antigen is expressed at a low level, or in the case of a metastatic tumor, the antigen profile may change between the first-occurring tumor and the metastatic tumor, and the immune evasion mechanism of tumor cells also partly explains the reason for the low success rate.

Desired vaccine antigens should be expressed exclusively or at least at increased levels in tumor cells. HER2/neu (hereinafter referred to as "HER2") is an epidermal growth factor receptor (EGFR) family (EGFR2), and the overexpression of EGFR is observed in many carcinomas. In particular, HER2 overexpression is observed in 15-30% of all breast cancer patients, and HER2 expression is known to be associated with recurrence and poor prognoses in breast cancer patients.

HER2 is a transmembrane protein having the amino acid sequence of SEQ ID NO: 1, and consists of an intracellular domain (ICD, HER2 aa 676-1255) having protein tyrosine kinase activity, a transmembrane domain (TM, HER2 aa 653-675), and an extracellular domain (ECM, HER2 aa 22-652).

HER2 in breast cancer has been studied quite extensively, and several immunogenic peptides have been discovered. For example, in the case of a peptide of amino acids 369-377 of the HER2 protein (nelipepimut-S [NeuVax; HER2 aa369-377; E75 (NP-S)]), immunotherapy such as loading into dendritic cells and re-injection has been attempted, but clinically significant therapeutic or protective effects have not been reported in women with advanced breast cancer. Moreover, in a recent large-scale phase 3 clinical trial, the clinical trial was discontinued because there was no difference in disease free survival (DFS) from the placebo group.

As examples of other HER2 peptide vaccines, there are AE37 (LRMK-GVGSPYVSRLLGICL: LRMK-HER2 aa 776-790), which is a HER2-ICD peptide vaccine, GP2 (HER2 aa 654-662), which is a HER2-TM peptide vaccine, and the like, and some findings of the effect of these vaccines on increasing survival rates have been reported. In addition, it has been reported in animal experiments that a vaccine against a HER2-ICD fragment (hereinafter referred to as "HER2-ICD") reduces the size of tumors compared to a vaccine against a HER2-ECD fragment (hereinafter referred to as "HER2-ECD"), but the results of direct application of a HER-2 ICD DNA vaccine to breast cancer patients have not been reported.

Vaccines for the purpose of treating diseases are, due to their characteristics, often repeatedly administered with a specific cycle of two or more times, and long-term immunogenicity using the immune system of a subject must be produced, and thus, a long period of time is required to identify the effects of vaccines.

However, when it is confirmed that, considering the characteristics of a cancer disease and the mechanism of action of a drug, a cancer vaccine for the treatment of or prevention of recurrence in breast cancer patients has no therapeutic effect after long-term observation, it is often that case that the disease has already worsened and there is no further treatment method. Therefore, it is most important to select in advance patients for whom the vaccine will have a therapeutic effect and to administer drugs thereto.

However, until now, nothing is known about the selection of vaccination targets for breast cancer treatment.

### [Cited References]

### [Patent Documents]

Korean Patent Registration No. 10-1572474 (published date: November 30, 2015)
US Patent Publication No. US 2002/0193329 (published date: December 19, 2002)

### [Non-Patent Document]

Breast Care 2016; 11:116-121
Clin Cancer Res; 25(14) July 15, 2019

### Disclosure

### Technical Problem

Therefore, as a result of having conducted research to address the above problems, the inventors of the present disclosure confirmed that a DNA vaccine composition including a plasmid including a nucleic acid sequence encoding HER2-ICD can stably increase immunogenicity against a HER2-ICD antigen in breast cancer patients without side effects, and discovered a patient group exhibiting long-term immunogenicity against the vaccine composition, thus completing the present disclosure.

It is an object of the present disclosure to provide a method for predicting the acquisition of immunogenicity against a DNA vaccine and the therapeutic efficacy thereof through a method of examining immunogenicity against a vaccine antigen (HER2-ICD antigen) before DNA vaccination.

Another object of the present disclosure is to provide a method for enriching a DNA vaccination target through a method for predicting the acquisition of immunogenicity against a DNA vaccine and the therapeutic efficacy thereof.

Another object of the present disclosure is to provide a method of providing information for selecting a patient who will acquire immunogenicity against a DNA vaccine and have therapeutic efficacy thereby, through the method of enriching a DNA vaccination target.

### Technical Solution

To achieve the above object, the present disclosure provides a method for predicting the acquisition of immunogenicity against a DNA vaccine and the therapeutic efficacy thereof through a method of examining immunogenicity against a vaccine antigen (HER2-ICD antigen) before DNA vaccination.

A DNA vaccination target (patient group) may be selected using a method for predicting responsiveness to the DNA vaccine.

### Advantageous Effects

A method for predicting the acquisition of immunogenicity against a DNA vaccine (HER2-ICD DNA vaccine) and the therapeutic efficacy thereof, according to the present disclosure, has the effects of predicting the acquisition of immunogenicity against a DNA vaccine and the therapeutic efficacy thereof before DNA vaccination without big burden to a patient, and selecting a vaccination target (patient group) who can acquire immunogenicity against a DNA vaccine and exhibit therapeutic efficacy thereby.

### Description of Drawings

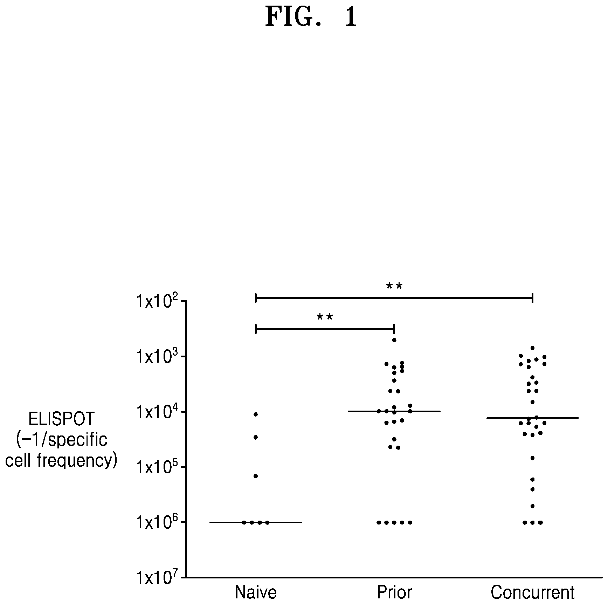

FIG. 1 illustrates immunogenicity against a HER2-ICD antigen prior to DNA vaccination at baseline on the basis of exposure to HER2-directed therapy.

### Best Mode

Hereinafter, the present disclosure will be described in more detail.

As used herein, the singular forms ("a," "an," and "the") include plural referents unless the context clearly indicates otherwise.

In describing and claiming certain features of the present disclosure, the following terminology will be used in accordance with the definitions described below unless indicated otherwise.

It is to be understood that, although aspects are described herein with the term "comprising," other similar aspects described in terms of "consisting of" and/or "consisting essentially of" are also provided.

The term "prevent" refers to success or a sign of success in preventing breast cancer recurrence/relapse when a patient in clinical remission is exampled by radiation, a physical method, or the like.

The term "no recurrence" means a state in which there is no recurrence of breast cancer or no recurrence of cancer in other sites when a patient in clinical remission is examined by radiation, a physical method, or the like.

Accordingly, the term "recurrence (relapse)" or "resurgence" is interchangeably used herein and refer to signs or symptoms of breast cancer or the onset of cancer in other sites after a period of amelioration, alleviation or remission by breast cancer treatment.

The terms "free of breast cancer," "disease free," "complete remission," or "No Evidence of Disease (NED)" mean that a patient is currently in clinical remission by standard medical therapy. In this case, this means that, although cancer cells may still be present in the body, the clinical signs, radiographic signs and symptoms of breast cancer are remarkably reduced or completely disappear on the basis of clinical diagnosis. Thus, remission is construed as including partial remission and complete remission.

The term "immunity (immunogenicity)" means the ability of a specific substance, such as an antigen, to induce an immune response in a subject. In the present disclosure, the immunogenicity refers to the ability to induce an immune response against a HER2-ICD antigen, and the immune response is measured by IFN-γ ELISPOT (stimulation of PBMCs isolated from a subject with the HER2-ICD antigen), but the disclosure is not limited thereto.

In an embodiment of the present disclosure, in a case in which the amount of IFN-γ produced through stimulation of peripheral blood mononuclear cells (PBMCs) by HER2-ICD is measured by enzyme-linked immuno-spot assay (ELISpot), when the production of HER2-ICD antigen-specific IFN-γ is more than twice compared to baseline, a HER2-ICD antigen-specific T cell response is increased, which is considered that immunogenicity has occurred after vaccination.

The term "immune response" refers to a physiological response by the immune system of a subject to an antigen, and in the present disclosure, the immune response refers to, but is not limited to, protective immunity against the HER2-ICD antigen as a result of such an immune response.

"Protective immunity" or "protective immune response" as used herein means that a subject initiates an immune response against the HER2-ICD antigen so that the immune system of the subject can target and destroy cells expressing the same antigen, thereby reducing cancer recurrence or cancer progression in the subject. In the present disclosure, protective immunity is conferred by T lymphocytes, but the disclosure is not limited thereto.

In the present disclosure, in a case where the amount of IFN-γ produced through stimulation of peripheral blood mononuclear cells (PBMCs) of a subject by HER2-ICD before (baseline)/after vaccine administration was measured by enzyme-linked immune-spot assay (ELISpot), when the production of HER2-ICD antigen-specific IFN-γ was two-fold or greater as compared to baseline, this was regarded as an increased HER2-ICD antigen-specific T cell response.

The term "booster" refers to a certain dose of an immunogen administered to a patient to enhance, prolong, or maintain protective immunity and to overcome the down-regulation of T-cell responses mediated by regulatory T-cells.

The term "pharmaceutically acceptable" refers to substances which are acceptable to a patient from a pharmacological/toxicological point of view regarding composition, formulation, safety, and the like, and the term "pharmaceutically acceptable carrier" refers to a medium that does not interfere with the effectiveness of the biological activity of the active ingredient(s) and is not toxic to a subject upon administration.

The term "about" may mean, when referring to a measurable value, such as weight, duration, or the like, variations of ±20%, ±10%, ±5%, ±1%, or ±0.1% from the specified value, but the disclosure is not limited thereto, and refers to a range that does not affect reactivity, including variations suitable for carrying out the described methods.

The term "patient" or "subject" refers to a living organism suffering from, treated for, or susceptible to a condition, such as breast cancer, in which a disease can be prevented or treated via administration of the vaccine composition of the present disclosure, and includes both humans and animals. Examples of the subject include, but are not limited to, mammals (e.g., mice, monkeys, horses, cows, pigs, dogs, and cats), and are preferably humans. In addition, "patient" or "subject" as used herein interchangeably includes breast cancer patients or breast cancer patients expressing HER2.

The term "administering" refers to introducing a vaccine composition into a subject by using any of a variety of methods and delivery systems known in the art to which the present disclosure pertains. Examples of administration routes include intravenous administration, intramuscular administration, subcutaneous administration, intraperitoneal administration, spinal administration, or other parenteral routes of administration, such as injection or infusion. The term "administration" as used herein refers to an intradermal administration method, generally by injection, and includes, but is not limited to, intravenous administration, intramuscular administration, intraarterial administration, intrathecal administration, or intralymphatic injection and infusion. Other parenteral routes include topical, epithelial, or mucosal routes of administration, such as intranasal, vaginal, rectal, or sublingual routes. In the present disclosure, "vaccine injection" to a subject, "treatment," and "vaccination" are used interchangeably.

The term "cancer immunotherapeutic agent" as used herein is a therapeutic agent that induces immune cells to selectively attack only cancer cells by stimulating the immune system by injecting an artificial immune protein into the body, unlike conventional anticancer agents that attack cancer itself, and is an anticancer agent with a mechanism that restores or strengthens the tumor recognition ability or destruction ability of the immune system to overcome the immunosuppression or immune evasion mechanism acquired by cancer cells. The cancer immunotherapeutic agent includes, but is not limited to, an immune checkpoint inhibitor, an immune cell therapeutic agent, and immune virus therapeutic agent.

The term "immune checkpoint inhibitor" as used herein is a type of cancer immunotherapeutic agent that serves to attack cancer cells by activating T cells by blocking the activation of an immune checkpoint protein involved in T cell inhibition, when some cancer cells use the immune checkpoint of T cells, which are immune cells in the body, to evade immunity, and examples thereof include, but are not limited to, a CTLA-4 inhibitor, a PD-1 inhibitor, a LAG-3 inhibitor, a TIM-3 inhibitor, a TIGIT inhibitor, and a VISTA inhibitor. In addition, the immune checkpoint inhibitor is an antagonist, and may be an antagonist antibody or a small molecule compound.

The term "therapy targeting HER2" refers to therapy by a mechanism that inhibits the activity of the HER2 protein, and refers to therapy by a drug that blocks the active domain of HER2. Specific drugs may include therapeutic antibodies, small molecules, antibody-drug conjugates (ADCs) and the like that have an antagonistic action against HER2. An example of a representative antagonist antibody is trastuzumab, but the disclosure is not limited thereto. In the present disclosure, "therapy targeting HER2" and "HER2-targeted therapy" are used interchangeably.

The composition disclosed herein is prepared as a DNA vaccine. In some embodiments of the present disclosure, the DNA vaccine includes a plasmid having a promoter, appropriate transcriptional and translational control elements, and a nucleic acid sequence encoding one or more polypeptides of the present disclosure. In some embodiments, the plasmid may further include an enhancement sequence, e.g., a sequence that enhances an expression level, intracellular targeting, and the like.

An antigen of the vaccine composition of the present disclosure is the intracellular domain (ICD) of aa 676-1255 of the HER2 gene (HER2-ICD). The polypeptide sequence of the HER2-ICD may have 100% homology to the amino acid sequence of SEQ ID NO: 2, or may have at least 99% homology, at least 95% homology, at least 90% homology, at least 85% homology, or at least 80% homology thereto. However, in the case of homology limited to the above specific value, immunogenicity against the HER2-ICD antigen due to vaccination has the same or equivalent effect as that of the sequence having 100% homology.

In some embodiments of the present disclosure, the DNA vaccine includes a plasmid vector including the nucleic acid sequence encoding HER2-ICD of the present disclosure. In some embodiments, the plasmid is a pUMVC3 (since the conventional name is "pNGVL3," the two names may be used interchangeably) plasmid. The nucleic acid sequence encoding HER2-ICD may have 100% homology to the nucleic acid sequence of SEQ ID NO: 3, or may have at least 99% homology, at least 95% homology, at least 90% homology, at least 85% homology, or at least 80% homology thereto. However, in the case of homology limited to the above specific value, immunogenicity against the HER2-ICD antigen due to vaccination has the same or equivalent effect as that of the sequence having 100% homology.

In some embodiments of the present disclosure, the DNA vaccine may additionally encode immunomodulatory molecules that modulate the resulting immune response, such as enhancing the potency of the vaccine, stimulating the immune system, or reducing immunosuppression.

In some embodiments of the present disclosure, a patient group to which the HER2-ICD DNA vaccine composition is administered is a patient group with stage 3 or 4 breast cancer, which expresses HER2. Each patient was in complete remission after standard breast cancer treatment was completed, or only stable bone only disease (SBO) was allowed in a patient group with stage 4 breast cancer.

Since the vaccine composition of the present disclosure is to induce and/or maintain protective immunity against breast cancer recurrence, the vaccine composition of the present disclosure may be administered to a patient according to any schedule suitable for inducing and/or maintaining a cytotoxic T cell response to the HER2-ICD antigen. For example, the vaccine composition as described and exemplified herein may be administered to a patient on a certain schedule as primary vaccination, followed by administration of a booster to induce and/or maintain protective immunity.

In some embodiments of the present disclosure, the vaccine composition may be administered to a patient once, twice or more times per month. The booster may be administered once or multiple times at regular intervals after completing the inoculation schedule, e.g., at intervals of one month, or may be administered once or multiple times over a period of 6 months or longer. In some embodiments of the present disclosure, the HER2-ICD vaccine was administered three times once a month as primary vaccination, but an additional booster may be inoculated after 6 months or 1 year.

In this regard, the booster may have the same dose and composition as the vaccine composition used in primary vaccination, and the dose and/or composition thereof may vary.

The term "dose" refers to the total amount of the active ingredient (HER2-ICD plasmid DNA) of the vaccine composition administered once to a subject. In some embodiments of the present disclosure, the HER2-ICD DNA vaccine composition was administered at selected doses of 10 µg, 100 µg, and 500 µg per arm (22-patient group), respectively for three arms. In each arm, an increase in immunogenicity against the HER2-ICD antigen by vaccination was observed, and all vaccine-related side effects in the respective arms were Grade 1 and Grade 2 (extremely low grades), and no serious Grade 3-5 side effects were found. With regard to the side effects, there was no statistically significant difference between the respective arms.

Surprisingly, immunogenicity against HER2-ECD could be increased in a patient group administered with the vaccine composition of the present disclosure. The DNA vaccine composition of the present disclosure is a vaccine using HER2-ICD as an antigen, and has a sequence different from that of HER2-ECD or a peptide, but immunoreactivity to HER2-ECD was also increased in patient groups administered with the vaccine composition of the present disclosure, particularly Arm 2 and Arm 3 patient groups.

Considering these effects, the vaccine composition of the present disclosure was confirmed to maintain long-term immunoreactivity to HER2-ICD even after vaccine administration is completed, and will have an excellent effect in preventing breast cancer recurrence or metastasis in a patient group in remission with breast cancer treatment.

In the vaccine composition of the present disclosure, the total amount of HER2-ICD plasmid DNA included in the vaccine administered once may be in a range of 10 µg to 1,000 µg, 20 µg to 1,000 µg, 30 µg to 1,000 µg, 40 µg to 1,000 µg, 50 µg to 1,000 µg, 60 µg to 1,000 µg, 70 µg to 1,000 µg, 10 µg to 900 µg, 20 µg to 900 µg, 30 µg to 900 µg, 40 µg to 900 µg, 50 µg to 900 µg, 60 µg to 900 µg, 70 µg to 900 µg, 10 µg to 800 µg, 20 µg g to 800 µg, 30 µg to 800 µg, 40 µg to 800 µg, 50 µg to 800 µg, 60 µg to 800 µg, 70 µg to 800 µg, 10 µg to 700 µg, 20 µg to 700 µg, 30 µg to 700 µg, 40 µg to 700 µg, 50 µg to 700 µg, 60 µg to 700 µg, 70 µg to 700 µg, 10 µg to 600 µg, 20 µg to 600 µg, 30 µg to 600 µg, 40 µg to 600 µg, 50 µg to 600 µg, 60 µg to 600 µg, 70 µg to 600 µg, 10 µg to 500 µg, 20 µg to 500 µg, 30 µg to 500 µg, 40 µg to 500 µg, 50 µg to 500 µg, 60 µg to 500 µg, 70 µg to 500 µg; 10 µg to 900 µg, 10 µg to 800 µg, 10 µg to 700 µg, 10 µg to 600 µg, 10 µg to 500 µg, 10 µg to 400 µg, 10 µg to 300 µg, 20 µg to 900 µg, 20 µg to 800 µg, 2 0 µg to 700 µg, 20 µg to 600 µg, 20 µg to 500 µg, 20 µg to 400 µg, 20 µg to 300 µg, 30 µg to 900 µg, 30 µg to 800 µg, 30 µg to 700 µg, 30 µg to 600 µg, 30 µg to 500 µg, 30 µg to 400 µg, 30 µg to 300 µg, 40 µg to 900 µg, 40 µg to 800 µg, 40 µg to 700 µg, 40 µg to 600 µg, 40 µg to 500 µg, 40 µg to 400 µg, 40 µg to 3 00 µg, 50 µg to 900 µg, 50 µg to 800 µg, 50 µg to 700 µg, 50 µg to 600 µg, 50 µg to 500 µg, 50 µg to 400 µg, or 50 µg to 300 µg.

When the total amount of HER2-ICD plasmid DNA included in the vaccine administered once is 1 µg or less, the desired immune response may not occur, and when the total amount of HER2-ICD plasmid DNA exceeds 1,000 µg, hypersensitivity reaction at the injection site or immune tolerance may occur and immunity may rather decrease.

In vaccination, predicting immune reactogenicity is the most important. Since vaccines generally require two or more repeated administrations and the purpose of vaccines is to acquire long-term immunogenicity, a long period of time is required to identify the efficacy of vaccines.

Particularly, in a therapeutic vaccine, the acquisition of immunogenicity against an antigen included in the vaccine and persistence thereof (long-term immunogenicity) are the most important because long-term immunogenicity is directly related to the therapeutic efficacy.

Specifically, in the case of cancer vaccines for treating cancer patients or preventing recurrence, there are many cases in which, when a vaccine is confirmed to have no efficacy after vaccination, there is no time to apply other treatment methods. Thus, it is the most important to pre-select and vaccinate patients on whom the vaccine can be expected to have therapeutic efficacy, i.e., a patient group who is likely to acquire immunogenicity against an antigen included in the vaccine and have long-term immunogenicity (therapeutic efficacy).

In the present disclosure, it was confirmed that, when there was almost no immunogenicity (immunity) against the HER2-ICD antigen in peripheral blood mononuclear cells (PBMCs) before HER2-ICD DNA vaccination, i.e., in the case of almost no immune response at baseline in an immune test for the HER2-ICD antigen, immunogenicity against the HER2-ICD antigen after vaccination was increased (more than twice) and the increased immunogenicity was maintained up to 12 months (long term follow-up: LTFU) after the completion of vaccination. However, in the case of patients who already have immunogenicity against the HER2-ICD antigen at baseline, immunogenicity against the HER2-ICD antigen after vaccination was often not increased more than twice compared to immunogenicity before vaccination (baseline) (not determined as being increased), and in many cases, even when the immunogenicity was increased, the increased immunogenicity was not maintained for a long period of time.

Although there may be various mechanisms by which immunogenicity against the HER2-ICD antigen may be acquired already prior to vaccination, immunogenicity against to the HER2-ICD antigen may also be acquired by exposure to HER2-directed therapy. In an embodiment of the present disclosure, it was confirmed that, although a patient group (Naive group) who had no experience of receiving HER2-directed therapy showed little immunogenicity against the HER2-ICD antigen at baseline, patient groups who had completed HER2-directed therapy (trastuzumab is prescribed in the present disclosure) (Prior treatment), or experienced exposure to HER2-directed therapy, such as receiving HER2-directed therapy during the vaccination period (concurrent treatment), showed increased immunogenicity against the HER2-ICD antigen at baseline.

Thus, it can be seen that it is preferable to select a patient group showing little immunogenicity against HER2-ICD at baseline as a subject for HER2-ICD DNA vaccination, wherein among these patients, many patients are not exposed to HER2-directed therapy, and it is preferable to select a patient group with low or almost no immunogenicity against the HER2-ICD antigen at baseline, even for patients who experienced HER2-directed therapy.

These results are also related to the persistence of vaccine DNA at the site of vaccination. In DNA vaccines, DNA (plasmid DNA) serves as a continuous source for producing the desired antigen by transfecting cells at the site of vaccine administration. Therefore, when DNA is continuously present at the site of vaccine administration for a long period of time, antigens can be continuously produced, which is thought to be helpful in maintaining antigen-specific immunity. In contrast, when plasmid DNA, which is a heterologous DNA carrier, persists for a long period of time in the patient's body, it may cause an immune response to the plasmid DNA itself in the subject.

In an embodiment of the present disclosure, as a result of separating skin tissue through biopsy of a site of vaccine administration one month after the vaccination was completed and amplifying vector DNA through PCR, vector DNA was detected in 31 patients and not detected in 21 patients. As a result of analyzing immunogenicity against the HER2-ICD antigen in PBMCs of each patient, it was confirmed that a 21-patient group in which vector DNA was not detected showed a tendency of increased immunoreactivity to the HER2-ICD antigen compared to patients in which vector DNA was detected, and in particular, immunoreactivity at LTFU (12 months after vaccination completion) showed a significant difference.

Surprisingly, more than half (61%) of patients with detected vaccine DNA experienced HER2-directed therapy during the vaccination period, and 76% of patients without detected vaccine DNA did not experience HER2-directed therapy. In addition, a statistically significant difference was shown between the two values.

These results indicate that the patient group with a long duration of vaccine DNA at the vaccination site and the patient group exposed to HER2-directed therapy, showing high immunogenicity against the HER2-ICD antigen before vaccination, show the same distribution, which can be seen as supporting the results that the less immunogenicity against the HER2-ICD antigen before vaccination mentioned above, the more favorable the enhancement of immunogenicity and securement of long-term immunogenicity by vaccination.

The vaccine composition disclosed herein may include, in addition to the plasmid including DNA encoding HER2-ICD, a pharmaceutically acceptable excipient, a carrier, a diluent, a buffer, a stabilizer, a preservative, an adjuvant, or other substances well known in the art to which the present disclosure pertains. These substances are non-toxic and do not interfere with the pharmaceutical activity of the active ingredient(s). A pharmaceutical carrier or a diluent may be, for example, an aqueous solution. The carrier or other substances that may be included in the vaccine composition of the present disclosure may be selected differently depending on administration route (e.g., oral, intravenous, dermal or subcutaneous, nasal, intramuscular, intradermal and intraperitoneal administration).

The vaccine composition of the present disclosure may include one or more "pharmaceutically acceptable carriers." These carriers typically include proteins, saccharides, polylactic acids, polyglycolic acids, polymeric amino acids, amino acid copolymers, sucrose, trehalose, and the like. Such carriers are well known in the art to which the present disclosure pertains. The vaccine composition may also contain diluents such as water, saline, and glycerol. In addition, the vaccine composition may include auxiliary substances such as a wetting agent or an emulsifying agent, and a pH buffer substance. Sterile pyrogen-free phosphate buffered saline, tromethamine (Tris) are representative carriers.

An aspect of the present disclosure relates to the vaccine composition as described above including one or more plasmid DNAs as an active ingredient, or a kit. The vaccine composition and the kit may be intended for use in a method of inducing an immune response in a subject or providing treatment, vaccinating, or immunotherapy, and the vaccine composition may be a vaccine or an immunotherapeutic composition. Such treatment or vaccination includes administering the vaccine composition of the present disclosure to a subject. The vaccine composition may be administered to a subject on a predetermined schedule.

The vaccine composition of the present disclosure may be lyophilized or in aqueous form, for example, an aqueous solution or a suspension. Liquid formulations of this type are ideal for injection as the composition can be administered directly in packaged form without the need for reconstitution in an aqueous medium. The vaccine composition may be provided in vials or may be provided in pre-filled syringes. Syringes may be supplied with or without a needle. Syringes contain a single dose, whereas vials may contain a single dose or multiple doses. In addition, the vaccine composition of the present disclosure may be administered using a microneedle.

In addition, the vaccine composition of the present disclosure may be formulated in a sustained-release vehicle or a depot preparation. Such long-acting formulations may be administered by inoculation or implantation (for example, subcutaneously or intramuscularly) or by injection. Thus, for example, the vaccine composition may be formulated with a suitable polymeric or hydrophobic material (for example, an emulsion in an acceptable oil) or an ion exchange resin, or as a sparingly soluble derivative (for example, a sparingly soluble salt). Liposomes and emulsions are well-known examples of delivery vehicles suitable for use as carriers.

The vaccine composition of the present disclosure may include an antimicrobial agent when packaged in multiple dose formats. The antimicrobial agent may be selected from 2-phenoxyethanol, parabens (methyl, ethyl, and propyl parabens), and the like. Any preservatives are preferably present at low levels. Preservatives may be added exogenously and/or may be a component of bulk antigens that are mixed to form a composition.

The vaccine composition of the present disclosure may be provided in the form of a kit including instructions and the like.

The vaccine composition of the present disclosure may include one or more adjuvants to increase the immunogenicity of the composition.

The suitable adjuvant includes an aluminum salt such as aluminum hydroxide or aluminum phosphate, but may be a salt of calcium, iron or zinc or may be an insoluble suspension of acylated tyrosine or acylated sugar, or may be selected from cationically or anionically derived sugars, but the disclosure is not limited thereto. In addition, the adjuvant may include cytokines used as immunomodulators.

The cytokines suitable for use as immunomodulators may be selected from interleukins (e.g., IL-1, IL-2, IL-4, IL-5, IL-6, IL-7, IL-12, and the like), macrophage colony stimulating factors (M-CSFs), tumor necrosis factors (TNFs), and granulocyte macrophage colony stimulating factors (GM-CSF), but the disclosure is not limited thereto.

In an embodiment of the present disclosure, GM-CSF may be included as an adjuvant.

The adjuvant may be added in an amount of 0.075 mg to 0.2 mg or 0.08 mg to 0.12 mg per dose.

The vaccine composition of the present disclosure may be administered in combination with one or more anticancer agents selected from chemotherapeutic agents, targeted anticancer agents, and cancer immunotherapeutic agents. One or more anticancer agents selected from among chemotherapeutic agents, targeted anticancer agents, and cancer immunotherapeutic agents that may be administered in combination with the vaccine composition of the present disclosure may be administered simultaneously or sequentially at a time interval, and may be selected according to an appropriate time and cycle.

In the present disclosure, chemotherapeutic agents are also called cytotoxic anticancer agents and chemical anticancer agents. Examples of the chemotherapeutic agent include, but are not limited to, gemcitabine, cytarabine, carboplatin (Paraplatin), cisplatin (Platinol, Platinol-AQ), crizotinib (Xalkori), cyclophosphamide (Cytoxan, Neosar), docetaxel (Taxotere), doxorubicin (Adriamycin), etoposide (Vepesid), 5-fluorouracil (5-FU), irinotecan (Camptosar), liposome-encapsulated doxorubicin (Doxil), methotrexate (Forlex, Mexate, amethopterin), paclitaxel (Taxol, Abraxane), topotecan (Hycamtin), trabectedin (Yondelis), vincristine (Oncovin, Vincasar PFS), or vinblastine (Velban).

In the present disclosure, the targeted anticancer agent may be, but is not limited to, at least one targeted therapeutic agent selected from the group consisting of trametinib, vemurafenib, alpelisib, dactolisib, gefitinib, erlotinib, lapatinib, sunitinib, sorafenib, crizotinib, dabrafenib, trastuzumab, cetuximab, bevacizumab, panitumumab, Ipilimumab, pertuzumab, tofacitinib, imatinib, bortezomib, ofatumumab, and alemtuzumab.

In the present disclosure, the caner immunotherapeutic agent may be, but is not limited to, any one immune checkpoint inhibitor selected from the group consisting of cytotoxic T lymphocyte-associated protein 4 (CTLA-4), programmed cell death protein 1 (PD-1), lymphocyte activation gene-3 (LAG3), T-cell immunoglobulin and mucin-domain containing-3 (TIM-3), T-cell immunoreceptor with IG and ITIM domain (TIGIT), and V-domain Ig suppressor of T cell activation (VISTA).

In the present disclosure, the composition may further include an anticancer adjuvant, and more preferably, the anticancer adjuvant may be, but is not limited to, a STimulator of InterferoN Gene (STING) agonist.

In some embodiments of the present disclosure, administration of the HER2-ICD DNA vaccine composition may be performed concurrently with treatment by other cancer therapeutic agents. As the cancer therapeutic agent, anticancer drugs such as fluorouracil, doxorubicin, and paclitaxel, which are standardly used for breast cancer treatment, Trastuzumab (Herceptin^{®}), which is an antibody therapeutic agent for HER2, and tamoxifen or bisphosphonate, which is a targeted therapeutic agent, may be used, but the disclosure is not limited thereto.

Unless otherwise indicated, all numbers used in the specification and claims, whether stated or not, are to be understood in all instances as being modified by the term "about." Also, the precise numerical values used in the specification and claims are to be understood as forming additional embodiments of the present disclosure. Efforts have been made to ensure the accuracy of numerical values disclosed in the examples. However, all measured values inherently may contain error values resulting from the standard deviations found in their respective measurement techniques.

### Mode for Invention

Hereinafter, the present disclosure will be described in further detail with reference to the following examples. These examples are provided only to explain the present disclosure in more detail, and it will be apparent to those of ordinary skill in the art that the scope of the present disclosure is not limited by these examples according to the gist of the present disclosure.

### Design and results of clinical trial (NCT00436254)

### Patient group

Patients who participated in the experiment were stage 3 or 4 breast cancer patients who met the following criteria. (1) Only patients who had completed standard therapy, were in complete remission, and had stable bone only disease were allowed for the case of stage 4 patients. (2) Cases determined as overexpressing the HER2 gene or protein in the primary tumor or metastasis stage through immunohistochemistry (IHC) or fluorescence in situ hybridization (FISH). (3) The ECOG performance score is 0 (SCOG Performance Score O). (4) White blood cell (WBC) ≥ 3000/mL, platelet count ≥ 100,000/mL, hematocrit ≥ 30; and must have normal kidney and liver functions. (5) No other immunosuppressive drug treatments such as chemotherapy or steroids for 30 days prior to clinical trial registration. (6) Left ventricular ejection fraction (LVEF) in the normal range.

Standard treatments such as bisphosphonate, anti-estrogen and Trastuzumab therapies were permitted during the vaccination period. All patients gave written informed consent in accordance with institutional and federal regulations. 66 patients were enrolled from May 2004 to August 2007, and 64 patients completed all three doses of vaccination. Two patients enrolled in Arm 1 discontinued the study due to disease progression after receiving one dose of the vaccine. Patients were followed for 10 years for the degree of organ toxicity according to federal regulations.

### Study design and treatment schedule

Patients participating in this experiment were sequentially assigned to one of the three pNGVL3-HER2-ICD groups (Arm 1: 10 µg, Arm 2: 100 µg, Arm 3: 500 µg) defined by vaccine dose. All vaccines are administeredintradermally (i.d.), and once a month for a total of three vaccines in the same draining lymph node site, divided into two injection sites for each vaccine dose. Patients received a tetanus toxoid vaccine as a positive control before the first vaccination. Patients simultaneously administered with trastuzumab had MUGA scans performed at baseline and 1 month after the last vaccine. Toxicity assessments were performed monthly, 3, 6 and 12 months after vaccine series were completed, and included physical examination, autoimmune serology, CBC, and a comprehensive metabolic panel. Toxicity was graded according to CTCAE v 3.0 and vaccine-related toxicity was defined as definitely, probably or possibly related adverse effects regardless of severity. Long-term side effects were assessed through an annual medical record review of surviving patients. PBMCs were collected at baseline and after the third vaccine dose (12 weeks) and 1, 3, 6, and 12 months after the final vaccination (corresponding to 16, 24, 36, and 60 weeks after clinical enrollment, respectively) to assess induced immune responses to HER2-ICD and HER2-ECD.

To evaluate the persistence of plasmid DNA, skin biopsies were obtained from the vaccine injection site 1 month and 3 months after the last vaccine administration. PBMCs were evaluated for the presence of plasmid DNA at baseline and 1 month after the last vaccine.

### Vaccine preparation

The HER2-ICD sequence (SEQ ID NO: 2) was inserted into pNGVL3 and identified by DNA sequencing. pNGVL3-HER2-ICD, which is a plasmid DNA vaccine, was amplified under GMP conditions and quantified, and vialed. Single-dose vials contain 10 µg, 100 µg or 500 µg plasmid DNA (pNGVL3-HER2-ICD) suspended in Tromethamine/EDTA as stabilization buffer (1.2 ml), as well as 100 µg GM-CSF in common. Vials were tested for microorganisms, sterility, and stability and stored between -25 °C and -10 °C at the University of Washington Investigational Drug Services Pharmacy in accordance with US Pharmacopoeia standards.

### Evaluation of antigen-specific T cell responses

PBMC samples from all patients were processed and cryopreserved using standardized methods proven to preserve lymphocyte function. All samples for each patient were simultaneously thawed and analyzed to reduce experimental variability. According to the previously known method of Disis et al. (J Clin Oncol. 2009 Oct 1; 27(28): 4685-92), a specific response to HER2 was measured by the IFN-γ ELISpot method stimulated by an antigen (stimulant), which will be described below. As the HER2-ICD antigen or HER2-ECD antigen (stimulant), a 15-amino-acid overlapping peptide pool (1 mg/mL) in which 11 amino acids overlap was used as an antigen.

Patients were considered to have increased HER2-ICD antigen-specific T cell responses when the production of HER2-ICD antigen-specific IFN-γ in PBMCs measured by the ELISpot method increased 2-fold from baseline after vaccination.

### Evaluation of persistence of plasmid DNA in skin and lymphocytes

Vaccines were administered between the two tattoo marks to serve as a consistent marker for post-vaccination skin biopsies. Biopsies were taken from designated inoculation sites (skin) at designated time periods. The isolated skin tissue was stored at 70 °C. Tissue and PBMC DNA were isolated using a DNA isolation kit, and vector DNA was amplified using a primer pair of PRDM36 primer [GACTAACAGACTGTTCCTTTCCATGG: coding primer] and PRDM52 primer [GCCAGAAGTCAGATGCTCAA: complementary primer].

### Patient group analysis and toxicity (adverse effect) analysis for each arm

Arm 1, Arm 2, and Arm 3 each consisted of 22 patients, and were administered intradermally with a vaccine composition comprising 10 µg, 100 µg, and 500 µg, respectively, of HER2-ICD plasmid DNA, and 100 µg of GM-CSF in common. Patient characteristics of each arm are shown in Table 1.

**[Table 1]**

| DNA vaccine dose | Arm 1 (10 µg) | | Arm 2 (100 µg) | | Arm 3 (500 µg) | |
|---|---|---|---|---|---|---|
| | No. | % | No. | % | No. | % |
| Age (~years): Median (range) | 50(38-68) | | 51(34-72) | | 53(42-77) | |

| Stage | | | | | | |
|---|---|---|---|---|---|---|
| III | 15 | 68 | 15 | 68 | 12 | 55 |
| IV (NED) | 3 | 14 | 4 | 18 | 7 | 32 |
| IV (SBO) | 4 | 18 | 3 | 14 | 3 | 14 |

| ER/PR status | | | | | | |
|---|---|---|---|---|---|---|
| ER+ and/or PR+ | 14 | 64 | 15 | 68 | 8 | 36 |
| ER-/PR- | 8 | 36 | 7 | 32 | 14 | 64 |

| Trastuzumab pretreatment | | | | | | |
|---|---|---|---|---|---|---|
| No | 4 | 18 | 2 | 9 | 2 | 9 |
| Yes | 18 | 32 | 20 | 91 | 20 | 91 |

| Simultaneous trastuzumab treatment | | | | | | |
|---|---|---|---|---|---|---|
| No | 14 | 64 | 12 | 55 | 9 | 41 |
| Yes | 8 | 36 | 10 | 45 | 13 | 59 |

| | | | | | | |
|---|---|---|---|---|---|---|
| ER: Estrogen Receptor PR: Progesterone Receptor NED: No Evidence of Disease SBO: Stable Bone-Only disease | | | | | | |

A total of 990 adverse effects were observed in all categories and 517 were considered vaccine-related (Table 2). The most common vaccine-related adverse events include injection site reactions (23%), fatigue (7%), and myalgia (7%). All vaccine-related toxicities were mostly grade 1 (89%) or grade 2 (11%), and there was no significant difference between the respective arms.

In terms of the safety of the DNA vaccine, since foreign DNA is administered to a subject, the risk of occurrence of an autoimmune reaction and/or insertion of the foreign DNA into the chromosomal DNA of the subject is very low, but exists. In this study, 16 autoimmune-related abnormal serological reactions were found in all the arms (Table 2). 11 patients showed ANA positivity during the vaccination period, and a transient decrease in C3 was observed in 5 patients.

All autoimmune-related toxicities were grade 1, anti-ds-DNA antibodies were not found in all patients, and no signs or occurrences of autoimmune diseases were found. DNA vaccine was not detected in circulating lymphocytes of all vaccinated patients.

Therefore, side effects caused by administration of the vaccine composition of the present disclosure are extremely low-grade and cannot be considered of concern in administration to a subject, and no signs of autoimmune diseases or occurrences of disease were observed. In addition, since insertion of the vaccine composition of the present disclosure into the genome of a subject was not observed, it is determined that there is no safety problem in clinical application.

**[Table 2]**

| DNA vaccine dose | total | | Arm 1 (10 µg) | | Arm 2 (100 µg) | | Arm 3 (500 µg) | |
|---|---|---|---|---|---|---|---|---|
| | No | % | No | % | No | % | No | % |
| Most frequent adverse events (n=188) | | | | | | | | |
| Injection site reaction | 117 | 23 | 38 | 7 | 48 | 9 | 31 | 6 |
| Cold-like reaction | 35 | 7 | 19 | 4 | 9 | 2 | 7 | 1 |
| Fatigue | 36 | 7 | 14 | 3 | 17 | 3 | 5 | 1 |

| Abnormal autoimmune response (n=16) | | | | | | | | |
|---|---|---|---|---|---|---|---|---|
| ANA | 11 | 2 | 4 | 1 | 3 | 1 | 4 | 1 |
| C3 | 5 | 1 | 3 | 1 | 1 | <1 | 1 | <1 |
| Anti-ds DNA | 0 | 0 | 0 | 0 | 0 | 0 | 0 | 0 |

| Grade of all adverse events (n=517) | | | | | | | | |
|---|---|---|---|---|---|---|---|---|
| 1 | 459 | 89 | 180 | 35 | 130 | 25 | 149 | 29 |
| 2 | 57 | 11 | 26 | 5 | 11 | 2 | 20 | 4 |
| 3 | 1 | <1 | 0 | 0 | 0 | 0 | 1 | <1 |
| 4 | 0 | 0 | 0 | 0 | 0 | 0 | 0 | 0 |
| 5 | 0 | 0 | 0 | 0 | 0 | 0 | 0 | 0 |

| | | | | | | | | |
|---|---|---|---|---|---|---|---|---|
| SBO: Stable Bone-Only disease ANA: anti-nuclear antibody C3: Complement 3 Anti-ds DNA: anti-double strand DNA antibody | | | | | | | | |

### Immunogenicity analysis for HER2-ICD before/after vaccination

To analyze immunogenicity (baseline) against the HER2-ICD antigen already possessed in each patient before vaccination, immunoreactivity to the HER-ICD antigen in PBMCs before vaccination was analyzed by the IFN-γ ELISPOT method. In addition, this value was used as a criterion for calculating a change in immunoreactivity to the HER2-ICD antigen after vaccination.

Patients before vaccination can be divided into: a Naive group without experience with HER2-directed therapy (trastuzumab is prescribed in this study); a Prior group that completed HER2-directed therapy before vaccination; and a Concurrent group receiving HER2-directed therapy during vaccination, depending on whether or not they experience HER2-directed therapy.

The immunogenicity against the HER2-ICD antigen by ELISPOT in each group is shown in FIG. 1, and was measured as 1:1000000 (1:10870 to 1:1000000) for the Naive group, 1:9707 (1:498 to 1:100000, p = 0.008) for the Prior group, and 1:12840 (1:691 to 1:000000, p=0.005) for the Concurrent group. 1:1000000, 1:9707, and 1:12840 of each group are the median values measured by the ELISPOT method. The values of the Naive group showed a statistical difference from those of the Prior and Concurrent groups, but there was no statistical difference between the values of the Prior and Concurrent groups.

Therefore, it can be seen that patients having experience with HER2-directed therapy have immunogenicity against the HER2-ICD antigen before vaccination, and specifically, have an about 10-fold increase in immunogenicity compared to patients without HER2-directed therapy experience.

Thereafter, immunoreactivity to the HER2-ICD antigen was analyzed in PBMCs isolated at 1 month and 12 months (Long Term Follow-up: LTFU) from the time of vaccination completion, from which it was confirmed that, surprisingly, in patients who did not show immunogenicity against the HER2-ICD antigen at baseline, not only did the immune response to the HER2-ICD antigen increase to a high level (more than 2-fold), but the level of immune response was maintained until LTFU. In addition, patients who did not show immunogenicity against the HER2-ICD antigen at baseline include most of the Naive patient group and some of the patient group receiving HER2-directed therapy.

Specifically, when reactivity to the HER2-ICD antigen was measured at baseline in 50 or more cells out of 10⁶ PBMCs by the ELISPOT method of the present disclosure (at least 1:20000), it can be considered that there is immunogenicity against the HER2-ICD antigen. In this case, there were few patients with more than a 2-fold increase in immunogenicity and/or long-term persistence even after vaccination with the HER2-ICD DNA vaccine.

Therefore, since a patient group with no or low immunogenicity against the HER2-ICD antigen is highly likely to acquire immunogenicity and exhibit long-term persistence due to the HER2-ICD DNA vaccine, for effective treatment, a patient group who has never received HER2-directed therapy or who has almost no immunogenicity against the HER2-ICD antigen at baseline before vaccination (specification based on clinical criteria) may be preselected (previously enriched) as the patient group to be vaccinated.

### Analysis of persistence of plasmid DNA at vaccination site

At 16 weeks after vaccination (1 month after the last vaccine administration), the presence of DNA vaccine in skin biopsies was analyzed by PCR amplification of vector DNA in which HER2-ICD was cloned.

Skin biopsies were obtained from 52 patients, and vector DNA was detected in 31 patients (60%) from among them. Vector DNA was detected in 61% of patients in Arm 1, 50% in Arm 2, and 73% in Arm 3.

As a result of follow-up of patients with and without DNA detection (Table 3), it was confirmed that not only did the patients with DNA detection have average low immunogenicity against the HER2-ICD antigen over time, but also had significantly low immunoreactivity against the HER2-ICD antigen at 60 weeks (12 months after vaccination) after vaccination (p=0.02).

**[Table 3]**

| Time point | DNA negative | | DNA positive | | p-value |
|---|---|---|---|---|---|
| | N | Median (IQR) | N | Median (IQR) | |
| Baseline | 21 | 70 (7, 555) | 31 | 81 (4, 243) | 0.65^{a} |
| Week 12-baseline | 19 | 4 (-99, 292) | 30 | -1 (-61, 44) | |
| Week 16-baseline | 20 | 16 (-25, 228) | 29 | 0 (-99, 17) | |
| Week 24-baseline | 19 | 0 (-390, 215) | 28 | -1 (-187, 87) | |
| Week 36-baseline | 18 | 25 (3, 301) | 28 | 14 (-86, 240) | |
| Week 60-baseline | 17 | 36 (-122, 417) | 27 | 0 (-31,249) | 0.02^{b} |

| | | | | | |
|---|---|---|---|---|---|
| IQR: interquartile range a: Wilcoxon rank test for baseline comparison b: Linear regression model for differences between baseline and ICD responses as a function of DNA persistence, treatment group, week, baseline HER2-ICD immune response, and age at enrollment. | | | | | |

Surprisingly, however, patients with vaccine DNA detected at week 16 were more likely to be exposed to HER2-directed therapy than patients without DNA detection. That is, 19 out of 31 patients with vaccine DNA detection received HER2-directed therapy (61%), and 5 out of 21 patients without DNA detection experienced HER2-directed therapy (24%) (p = 0.008).

These results indicate that the patient group with a long duration of vaccine DNA at the vaccination site and the patient group exposed to HER2-directed therapy, showing high immunogenicity against the HER2-ICD antigen before vaccination, show the same distribution, which can be seen as consistent with the results that the less immunogenicity against the HER2-ICD antigen before vaccination mentioned above, the more suitable it is to enhance immunogenicity and secure long-term immunogenicity by vaccination.

### Sequence List Text

### Sequence information

SEQ ID NO: 1 (HER2 full-length sequence) 
YLVPQQGFFCPDPAPGAGGMVHHRHRSSSTRSGGGDLTLGLEPSEEEAPRS
PLAPSEGAGSDVFDGDLGMGAAKGLQSLPTHDPSPLQRYSEDPTVPLPSETDGYVAP
LTCSPQPEYVNQPDVRPQPPSPREGPLPAARPAGATLERPKTLSPGKNGVVKDVFAF
GGAVENPEYLTPQGGAAPQPHPPPAFSPAFDNLYYWDQDPPERGAPPSTFKGTPTA
ENPEYLGLDVPV
SEQ ID NO: 2 (HER2-ICD amino acid sequence) 
MKRRQQKIRKYTMRRLLQETELVEPLTPSGAMPNQAQMRILKETELRKVKVLG
SGAFGTVYKGIWIPDGENVKIPVAIKVLRENTSPKANKEILDEAYVMAGVGSPYVSRLL
GICLTSTVQLVTQLMPYGCLLDHVRENRGRLGSQDLLNWCMQIAKGMSYLEDVRLVH
RDLAARNVLVKSPNHVKITDFGLARLLDIDETEYHADGGKVPIKWMALESILRRRFTHQ
SDVWSYGVTVWELMTFGAKPYDGIPAREIPDLLEKGERLPQPPICTIDVYMIMVKCWMI
DSECRPRFRELVSEFSRMARDPQRFVVIQNEDLGPASPLDSTFYRSLLEDDDMGDLV
DAEEYLVPQQGFFCPDPAPGAGGMVHHRHRSSSTRSGGGDLTLGLEPSEEEAPRSP
LAPSEGAGSDVFDGDLGMGAAKGLQSLPTHDPSPLQRYSEDPTVPLPSETDGYVAPL
TCSPQPEYVNQPDVRPQPPSPREGPLPAARPAGATLERAKTLSPGKNGVVKDVFAFG
GAVENPEYLTPQGGAAPQPHPPPAFSPAFDNLYYWDQDPPERGAPPSTFKGTPTAE
NPEYLGLDVPV
SEQ ID NO: 3 (HER2-ICD nucleotide sequence)

GGGACCTGGTGGATGCTGAGGAGTATCTGGTACCCCAGCAGGGCTTCTTCTGTCC
AGACCCTGCCCCGGGCGCTGGGGGCATGGTCCACCACAGGCACCGCAGCTCATC
TACCAGGAGTGGCGGTGGGGACCTGACACTAGGGCTGGAGCCCTCTGAAGAGGA
GGCCCCCAGGTCTCCACTGGCACCCTCCGAAGGGGCTGGCTCCGATGTATTTGAT
GGTGACCTGGGAATGGGGGCAGCCAAGGGGCTGCAAAGCCTCCCCACACATGAC
CCCAGCCCTCTACAGCGGTACAGTGAGGACCCCACAGTACCCCTGCCCTCTGAGA
CTGATGGCTACGTTGCCCCCCTGACCTGCAGCCCCCAGCCTGAATATGTGAACCA
GCCAGATGTTCGGCCCCAGCCCCCTTCGCCCCGAGAGGGCCCTCTGCCTGCTGC
CCGACCTGCTGGTGCCACTCTGGAAAGGGCCAAGACTCTCTCCCCAGGGAAGAAT
GGGGTCGTCAAAGACGTTTTTGCCTTTGGGGGTGCCGTGGAGAACCCCGAGTACT
TGACACCCCAGGGAGGAGCTGCCCCTCAGCCCCACCCTCCTCCTGCCTTCAGCCC
AGCCTTCGACAACCTCTATTACTGGGACCAGGACCCACCAGAGCGGGGGGCTCCA
CCCAGCACCTTCAAAGGGACACCTACGGCAGAGAACCCAGAGTACCTGGGTCTGG
ACGTGCCAGTGTAA

## Claims

1. A method for predicting the reactivity of a HER2-ICD DNA vaccine, comprising examining immunogenicity against a HER2-ICD antigen in peripheral blood mononuclear cells (PBMCs) of a patient to be vaccinated with a HER2-ICD (intracellular domain) DNA vaccine, before vaccination.

2. The method of claim 1, wherein the patient to be vaccinated with the HER2-ICD DNA vaccine is a cancer patient expressing HER2.

3. The method of claim 2, wherein the patient to be vaccinated with the HER2-ICD DNA vaccine has no experience with HER2-directed therapy before vaccination.

4. The method of claim 3, wherein the HER2-directed therapy is by a pharmaceutical composition comprising a therapeutic antibody against HER2.

5. The method of claim 4, wherein the therapeutic antibody against HER2 comprises trastuzumab.

6. A method for selecting a patent to be administered with a HER2-ICD DNA vaccine, by the method of any one of claims 1 to 5.

7. The method of any one of claims 1 to 5, wherein the HER2-ICD DNA vaccine comprises a plasmid vector comprising a nucleotide sequence encoding a HER2-ICD domain having SEQ ID NO: 2 or at least 80% homology thereto.

8. The method of claim 7, wherein the nucleotide sequence is SEQ ID NO: 3 or has at least 80% homology thereto.
